# EUROPEAN PATENT SPECIFICATION

(11) **EP 1 027 088 B1**
(45) Date of publication and mention of the grant of the patent: **15.09.2004**
(21) Application number: 98956111.3
(22) Date of filing: 16.10.1998
(51) Int. Cl.: A61M 25/00

(54) **IMPROVED INTRA-AORTIC BALLOON CATHETER**
INTRA-AORTALER BALLONKATHETER
CATHETER A BALLONNET INTRA-AORTIQUE AMELIORE

(30) Priority: 27.10.1997 US 958004
(43) Date of publication of application: 16.08.2000
(73) Proprietor: DATASCOPE INVESTMENT CORP., Montvale, N.J. 07645 (US)
(72) Inventor: MIKSZA, Anthony, Bethlehem, PA 18017 (US); LAKSIN, Olga, Scotch Plains, NJ 07076 (US); YANG, Hubert, Nutley, NJ 07110 (US); FRISCH, Frank, Succasunna, NJ 07876 (US); CHENEY, Beth, Warwick, NY 10990 (US); LUCAS, John, Sparta, NJ 07871 (US); LESCHINSKY, Boris, Waldwick, NJ 07643 (US); SCHOCK, Robert, Sparta, NJ 07871 (US)
(74) Representative: Harrison, Michael Charles
(86) International application number: PCT/US1998/021885
(87) International publication number: WO 1999/021604

(56) References cited:
- EP-A- 0 380 102
- EP-A- 0 638 327
- WO-A-95/17219
- WO-A-97/18005
- US-A- 4 362 150
- US-A- 5 456 665
- US-A- 5 496 276
- US-A- 5 514 073

## Description

### BACKGROUND OF THE INVENTION

### Technical Field

The invention relates to an intra-aortic balloon catheter. More particularly, the invention relates to a balloon which is inserted into the aorta of a patient to augment the pumping action of the patient's heart.

### Background Art

Intra-aortic balloon (IAB) catheters are used in patients with left heart failure to augment the pumping action of the heart. The catheters, approximately 1 meter long, have an inflatable and deflatable balloon at the distal end. The catheter is typically inserted into the femoral artery and moved up the descending thoracic aorta until the distal tip of the balloon is positioned just below or distal to the left subclavian artery. The proximal end of the catheter remains outside of the patient's body. A passageway for inflating and deflating the balloon extends through the catheter and is connected at its proximal end to an external pump. The patient's central aortic pressure is used to time the balloon and the patient's ECG may be used to trigger balloon inflation in synchronous counterpulsation to the patient's heart beat.

Intra-aortic balloon therapy increases coronary artery perfusion, decreases the workload of the left ventricle, and allows healing of the injured myocardium. Ideally, the balloon should be inflating immediately after the aortic valve closes and deflating just prior to the onset of systole. When properly coordinated, the inflation of the balloon raises the patient's diastolic pressure, increasing the oxygen supply to the myocardium; and balloon deflation just prior to the onset of systole lowers.the patient's diastolic pressure, reducing myocardial oxygen demand.

Intra-aortic balloon catheters may also have a central passageway or lumen which can be used to measure aortic pressure. In this dual lumen construction, the central lumen may also be used to accommodate a guide wire to facilitate placement of the catheter and to infuse fluids, or to do blood sampling.

Typical dual lumen intra-aortic balloon catheters have an outer, flexible, plastic tube, which serves as the inflating and deflating gas passageway, and a central tube therethrough formed of plastic tubing, stainless steel tubing, or wire coil embedded in plastic tubing. A polyurethane compound is used to form the balloon.

All IAB catheters have two opposing design considerations. On the one hand, it is desirable to make the outer diameter of the entire catheter as small as possible: to facilitate insertion of the catheter into the aorta, maximizing blood flow past the inserted catheter, and to allow for the use of a smaller sheath to further maximize distal flow. On the other hand, however, it is desirable to make the inner diameter of the outer tube as large as possible because a large gas path area is required to accomplish the rapid inflation and deflation of the balloon. As a result of these opposing design considerations there is a need for a smaller catheter with a larger gas path area. Furthermore, there is also a need for a catheter demonstrating a high kink resistance.

One method of making the outer diameter of the wrapped balloon portion of the catheter as small as possible is to wrap the balloon in its deflated state as tightly as possible around the inner tube. Wrapping the balloon tightly, however, has posed a number of difficulties. First, it is difficult to wrap the balloon tightly because of the friction between contacting surfaces of the balloon. Second, contacting surfaces of a tightly wrapped balloon may stick upon initial inflation. Therefore, the need exists for a balloon which can be more easily wrapped tightly and which does not stick upon initial inflation. Furthermore, the need also exists for a retainer to maintain the tight wrapping of the balloon until the catheter is ready to be inserted.

Retainers presently on the market are generally tubes which slide over the balloon and thereby maintain the balloons tight wrapping. A tight fit between the wrapped balloon and the inner wall of the retaining tube is necessary. To accomplish this, however, if the fit is too tight the balloon may not be easily removed from the retaining tube without damaging or unwrapping the balloon. Therefore, the need exists for an easily removable retainer that can maintain a tight balloon wrapping.

A second method of making the outer diameter of the wrapped balloon portion of the catheter as small as possible is to decrease the size of the inner tube. A plastic inner tube, however, cannot be made small enough and still maintain the required stiffness and pressure transmitting qualities. US Patent No. 5,456,665 discloses an IAB having an inner tube made from Nitinol, a kink-resistant metal alloy manufactured and sold by Rayehem Corp. Use of Nitinol for the inner tube is desirable because a smaller diameter tube can be used while still maintaining the necessary stiffness.

One disadvantage of using Nitinol for the entire length of the inner tube, as disclosed by US Patent No. 5,456,665 is that Nitinol is a relatively expensive material. Accordingly, in order to produce a less costly catheter Nitinol should be used sparingly. If the gas flow path area can be increased using an alternated method aside from decreasing the size of the inner tube then Nitinol would only be necessary for the portion of the inner tube enveloped by the balloon and not for the portion of the inner tube disposed within the outer tube. Therefore, the need exists for a catheter having a larger gas path area, a smaller outer diameter, and a smaller unwrapped balloon diameter. The present invention provides these properties by having an inner tube with Nitinol properties only in the portion enveloped by the balloon.

US-A-5 514 073 discloses an IAB catheter having the features defined in the preamble of claim 1. In this document, a single continuous inner tube is fixed to the inner wall of the catheter tube. The gas for balloon inflation flows through the large outer lumen of the catheter tube to allow rapid inflation and deflation. The inner tube is made from a material which allows it to be attached or formed integrally with the inner wall of the catheter tube. Polyurethane, polyvinyl chloride, polyethylene and nylon are suggested as suitable materials.

EP-A-0 638 327 discloses a balloon dilatation catheter used for accessing the coronary arteries of a human. The catheter has two lumens, whereby the smaller one is used to supply a gas flow to the dilatation balloon and the larger one is used for a guidewire. The larger lumen portion of the catheter is divided into two segments which are attached together. The first segment is an intermediate segment formed of plastic material, such as high density polyethylene and the second segment is a distal segment formed of plastic material such as low density polyethylene so as thereby to be more flexible than the intermediate segment.

The need also exists for a newly designed tip which will track the guide wire easily, and thereby facilitate insertion. The tip is the most distal portion of the catheter. As a result of its location on the catheter, it has an appreciable impact on the guide wire tracking capabilities of the entire catheter.

The need further exists for a retainer that can easily and economically be attached to a catheter packaging tray. The retainer is removably attached to the packaging tray for two primary purposes. First, the retainer must be secured to the packaging tray for storage and transportation. Second, the retainer must be anchored to the packaging tray so that the balloon membrane can be removed from the retainer by simply pulling the catheter away from the retainer. One packaging attachment means currently on the market involves "U" shaped clips which snap over the retainers into grooves in the packaging tray. This packaging attachment means has a number of disadvantages. First, manufacture of the "U" shaped clips separately from the packaging tray increases packaging production costs. Second, the "U" shaped clips can be misplaced since they are not attached to the packaging tray. Third, the "U" shaped clips work properly only with retainers having robust flanges. A packaging attachment means incorporating "U" shaped clips works as follows: As the catheter is pulled away from the packaging material the retainer slides through the "U" shaped clip until the flange of said retainer, which cannot fit through the "U" shaped clip, is stopped by the "U" shaped clip. At this point, since the retainer is prevented from moving in the direction of the pull, the balloon membrane slides out of the proximal end of the retainer and the retainer remains in the packaging. If the retainer does not have a robust flange then it will slide right through the "U" shaped clip when the catheter is being pulled out of the packaging and the retainer will remain on the balloon membrane. Therefore, the third disadvantage of the packaging means incorporating a "U" shaped clip is that if it is desired to make the retainer from a nonthermoformable material, such as PTFE, which cannot easily accommodate a flange-like shaped end, such a retainer cannot be used in conjunction with a "U" shaped clip.

There is also a need for a catheter which minimizes the amount of diffusion of gas or fluid from the outer lumen into the inner lumen. Any adulteration of the fluid within the inner lumen may corrupt blood pressure readings. Typically, the outer lumen of an IAB catheter contains a gas, such as Helium, and the inner lumen contains an incompressible column of fluid, such as saline, used for blood pressure measurements. The distal end of the saline column is in contact with the patient's blood and the proximal end of the column is in contact with a pressure transducer. The pressure reading of the pressure transducer correlates to the blood pressure of the patient. Any diffusion of a gas into the inner lumen increases the compressibility of the saline column thus affecting the blood pressure reading. Therefore, there is a need for a catheter designed to minimize the diffusion of gas or fluid from the outer lumen into the inner lumen.

While the IAB catheters presently on the market may be suitable for the particular purpose employed, or for general use, they would not be as suitable for the purposes of the present invention as disclosed hereafter.

### SUMMARY OF THE INVENTION

It is an object of the invention to produce an improved IAB catheter having a co-lumen extrusion for the catheter body which maximizes the gas flow path area and reduces the amount of diffusion of fluid or gas from the outer lumen into the inner lumen.

It is another object of the invention to produce an improved IAB catheter having the portion of the inner tube, which extends into the balloon, made of Nitinol.

It is still another object of the invention to produce an improved IAB catheter having a MDX (MDX is a Dow Corning trademark for the descriptive name of the product, medical silicone) coated balloon membrane which can be wrapped tightly and not stick upon inflation.

The invention is an improved intra-aortic balloon catheter with a balloon membrane as defined in claim 1. Certain preferred features of the invention are defined in the dependent claims. The co-lumen tube material is preferably 0-70% by weight a PELLETHANE material having a hardness of 75D, 30-60% by weight a PELLETHANE material having a hardness of 65D, and 0-50% by weight a PELLETHANE material having a hardness of 55D. The tip preferably has a bulbous portion and is preferably made from ESTANE material group 58887 and preferably has a hardness less than or equal to 90A so as to allow for better guide wire tracking. The balloon membrane is preferably coated with a silicone liquid, such as MDX, and has a double-wall thickness within a range of 0.0001524 mm (0.006 inches) to 0.0002032 m (0.008 inches) so as to allow for a tighter wrap of the balloon membrane about the Nitinol inner tube. A retainer for the catheter may be made from a low friction PTFE material, such as TEFLON, so as to allow for easy removal of the tightly wrapped balloon membrane. The retainer may also have wings which snap under horizontal ribs and fit between vertical ribs in an improved packaging tray and thereby secure the retainer within said improved packaging tray.

To the accomplishment of the above and related objects the invention may be embodied in the form illustrated in the accompanying drawings. Attention is called to the fact, however, that the drawings are illustrative only. Variations are contemplated as being part of the invention, limited only by the scope of the claims.

### BRIEF DESCRIPTION OF THE DRAWINGS

In the drawings, like elements are depicted by like reference numerals. The drawings are briefly described as follows.

FIG 1 is a plane view of a prior art intra-aortic balloon catheter.

FIG 1A is a transverse cross section of the prior art catheter of FIG 1 taken along lines 1A-1A.

FIG 2 is a plane view of an improved intra-aortic balloon with a portion of its inner tube made of Nitinol.

FIG 2A is a transverse cross section of the improved catheter of FIG 2 taken along lines 2A-2A.

FIG 3A is a first profile of an improved tip having a bulbous portion.

FIG 3B is a second profile of an improved tip having a bulbous portion.

FIG 3C is a third profile of an improved tip having a bulbous portion.

FIG 3D is a fourth profile of an improved tip having a bulbous portion.

FIG 3E is a fifth profile of an improved tip having a "J" or hook portion.

FIG 4A is a perspective view of an improved catheter whose Nitinol and polyurethane inner tube portions were attached using a first method.

FIG 4B is a longitudinal view of the improved catheter of FIG 4A.

FIG 5 is a longitudinal cross sectional view of an improved catheter, whose Nitinol and polyurethane inner tube portions were attached using a second method.

FIG 6 is a longitudinal cross section of the improved catheter of FIG 2, having a Nitinol tube with a spiral cut.

FIG 7A is a longitudinal cross section of a prior art retainer packaged within a prior art packaging tray.

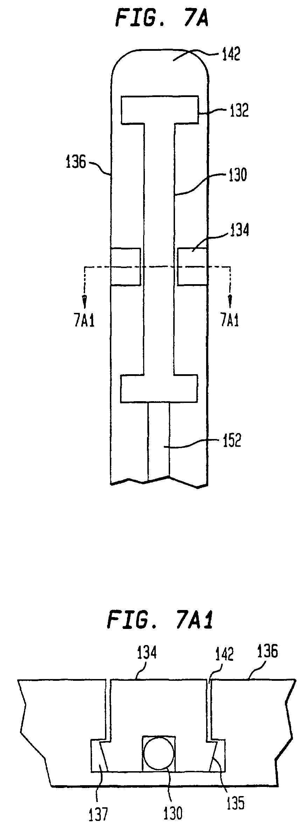

FIG 7A-1 is a transverse cross section of the prior art packaging tray taken along line 7A-7A in FIG 7A.

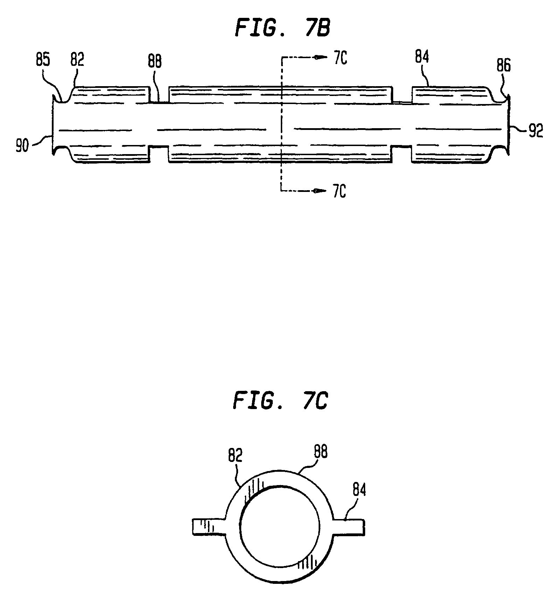

FIG 7B is a plane view of an improved retainer.

FIG 7C is a transverse cross sectional view of the improved retainer of FIG 7B.

FIG 7D is a plan view of the improved retainer packaged within an improved packaging tray.

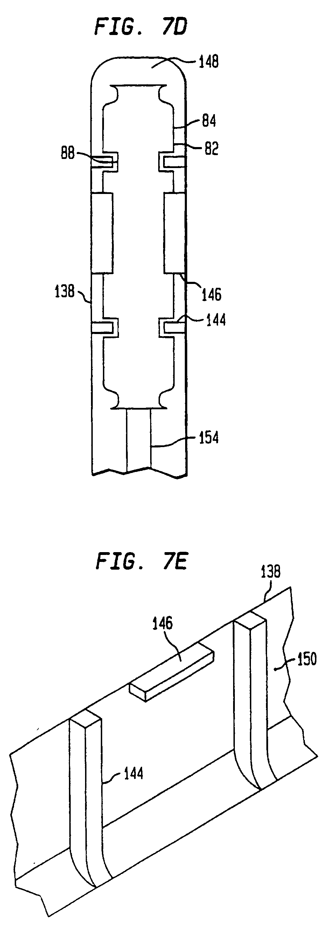

FIG 7E is a perspective view of one side of a channel in the improved packaging tray in which the improved retainer is stored.

### DETAILED DESCRIPTION OF THE PREFERRED EMBODIMENTS

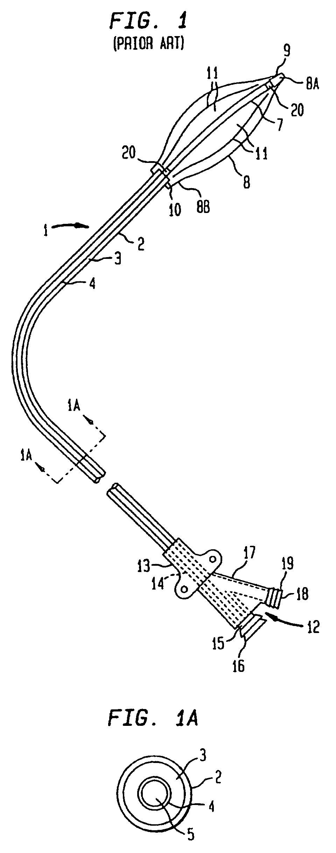

The general structure of an intra-aortic balloon catheter is best described in relation to FIGS 1 and 1A which illustrate a dual-lumen prior art intra-aortic balloon catheter. The catheter 1 is constructed of a clear plastic outer tube 2 forming a gas passageway lumen 3; and another clear plastic central tube 4 disposed within outer tube 2 and creating a central passageway or lumen 5 as may best be seen in FIG 1A.

A balloon 8 is disposed at the distal end of the catheter 1. The distal portion 7 of the central tube 4 extends beyond the distal end 10 of outer tube 2. The distal end 8A of the balloon 8 is attached to a tip 9 formed on the distal end 7 of central tube 4. The proximal end 8B of the balloon 8 is attached to the distal end 10 of the outer tube 2. The distal portion 7 of the central tube 4 supports the balloon 8. Said distal portion 7 must have sufficient strength to prevent inversion of the balloon 8 as it inflates and deflates under aortic pressure, but at the same time, be flexible enough to be safely inserted through an introducer sheath, moved through the arterial tree, and maintained in the thoracic aorta.

The balloon 8 is formed of a nonthrombogenic flexible material, such as polyurethane, and may have folds 11 formed as a result of wrapping the balloon 8 about the central tube 4 to ease insertion of the catheter 1. Radio-opaque bands 20 at the distal end of the catheter 1 aid in positioning the balloon 8 in the descending aorta.

Inflation and deflation of the balloon 8 is accomplished through the central passageway lumen 3. The central passageway or lumen can accommodate a guide wire for placement or repositioning of the catheter 1. When the guide wire is not disposed in the central lumen 5, the central lumen 5 may be used for measuring blood pressure in the descending aorta. This pressure measurement may be used to coordinate the inflation and deflation of the balloon 8 with the pumping of the heart, however, use of the patient's ECG is preferred. Additionally, the central lumen 5 may be used to infuse liquids into the descending aorta, or to sample blood.

At the proximal end 12 of the catheter 1 a hub 13 is formed on the proximal end 14 of the outer tube 2. The central passageway or lumen 5 extends through the hub 13 and a connector 16 is provided at the proximal end 15 (or exit) of the central passageway or lumen 5. Measurement of aortic pressure and blood sampling may be done through the proximal end 15 of the central passageway 5.

The proximal end 18 of the center passageway lumen 3 exits through a side arm 17 of the hub 13 on which is provided a connector 19. The proximal end 18 of the center passageway lumen 5 may be connected to an intra-aortic balloon pump.

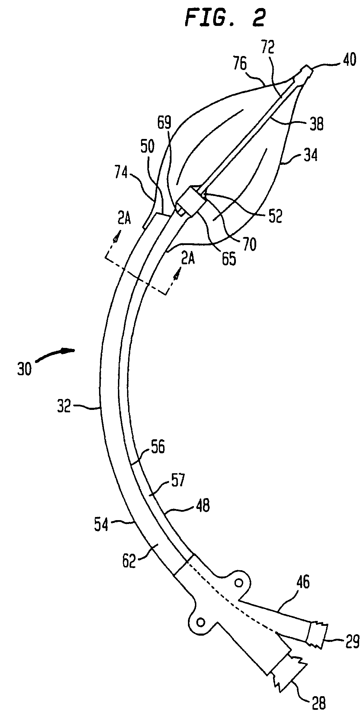

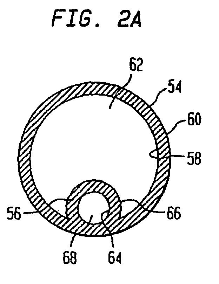

FIG 2 illustrates an embodiment of the improved IAB catheter 30. Said catheter 30 comprises a co-lumen extruded tube 32, a balloon membrane 34, a Nitinol inner tube 38, a tip 40, and a connector 46. The co-lumen extruded tube 32 has a proximal end 48, a first distal end 50, a second distal end 52, an outer tube portion 54, and an inner tube portion 56. The connector 46 has a first proximal end 28 and a second proximal end 29.

Nitinol is used as the material for the Nitinol inner tube 38 because it is desired to reduce the diameter of the wrapped balloon membrane 34 so as to allow for the use of a smaller sheath. The Nitinol inner tube 38 can be reduced in diameter to the appropriate size to accommodate a smaller sheath and yet still maintain the required stiffness.

FIG 2A illustrates a cross section of the co-lumen extruded tube 32 shown in FIG 2 taken along lines 2A-2A. The outer tube portion 54 of said co-lumen extruded tube 32 has an inner wall 58 and an outer wall 60. The inner tube portion 56 has an inner wall 64 and an outer wall 66. The inner wall 58 of the outer tube portion 54 and the outer wall 66 of the inner tube portion 56 define an outer lumen 62 passageway. Said outer lumen 62 extends from the first proximal end 28 of the connector 46 to the first distal end 50 of the co-lumen extruded tube 32. The inner wall 64 of the inner tube portion 56 defines a first inner lumen 68. The first inner lumen 68 extends from the second proximal end 29 of the connector 46 to the second distal end 52 of the co-lumen extruded tube 32. The inside wall of the Nitinol inner tube 38, as illustrated in FIG 2, defines a second inner lumen 69 which communicates with the first inner lumen 68. The first inner lumen 68 and the second inner lumen together form an inner lumen 57.

The inner lumen 57, as illustrated in FIG 2, extends from the second proximal end 29 of the connector 46 to the tip 40. The inner tube portion 56 has a distal end portion 65 which extends beyond the first distal end 50 of the co-lumen extruded tube 32. Said distal end portion 65 is attached to a Nitinol inner tube 38. The inner diameter of the inner tube portion 56 is larger than the outer diameter of the Nitinol inner tube 38. Said Nitinol inner tube 38 has a proximal end 70 and a distal end 72. The proximal end 70 is attached to the distal end portion 65 of the inner tube portion 56. A radio-opaque tip 40 is attached to the distal end 72 of the Nitinol inner tube 38. A balloon membrane 34 having a proximal end 74 and a distal end 76 is attached at its proximal end 74 just proximal to the first distal end 50 of the co-lumen extruded tube 32 and is attached at its distal end 76 to the tip 40. The balloon membrane 34 envelopes the Nitinol inner tube 38. Note that the first distal end 50 of the co-lumen extruded tube 32 is cut at an angle to the axis of the catheter 30 to facilitate gas flow into the balloon.

Note further that the co-lumen extruded tube 32 is more effective in preventing diffusion of gas or fluid from the outer lumen 62 into the inner lumen 57 than typical prior art catheters having central tubes because there is more material between the two lumens. The material separating the two lumens acts as a diffusion barrier. The fact that the inner lumen 57 is embedded in the wall of the co-lumen extruded tube 32 forces the fluid or gas either to diffuse through the small portion of the inner tube portion 56 that is not embedded in the co-lumen extruded tube 32 wall or to diffuse through the wall. The extra distance and material which the fluid or gas has to overcome before entering the inner lumen 57 reduces the diffusion rate from the outer lumen 62 into the inner lumen 57.

It is very important to wrap the balloon membrane 34 as tightly as possible so as to make the distal end of the catheter 30 as small as possible for easy insertion. The balloon membrane 34 should have a double-wall thickness ranging between 0.0001524 m (0.006 inches) and 0.000254 m (0.010 inches), preferably between 0.0001524 m and 0.0002032 m (.006 and .008 inches), for maximum wrapping ability. Furthermore, the balloon membrane 34 should be coated with an anti-friction non-stick coating such as medical silicone liquid(a descriptive name), preferably MDX grade 44159 medical silicone (MDX is a trademark of Dow Corning). A coating of MDX serves to reduce the coefficient of friction of the balloon membrane 34. Reducing the coefficient of friction serves two purposes: First, it decreases the friction between contacting portions of the balloon membrane 34 during wrapping, and thus, facilitates wrapping. Second, it minimizes sticking of contacting portions of the wrapped balloon membrane 34 during initial expansion.

Once the balloon membrane 34 is wrapped it is important to retain the tight wrap until the catheter 30 is ready to be inserted. The tight wrap is maintained by creating a vacuum within the balloon membrane while placing the wrapped balloon membrane within a membrane retainer. If the balloon membrane is held too tightly by the retainer, however, the balloon membrane may be damaged or may not unwrap upon removal of the retainer. The improved IAB, herein disclosed, uses a retainer made of PTFE (PTFE is the descriptive name of the material, it is sold under the name TEFLON). The PTFE retainer is used because it has a low coefficient of friction and thus allows for easy removal of the wrapped balloon membrane 34.

FIG 7A illustrates a longitudinal cross section of a prior art retainer 130 having flanged ends 132 secured by a "U" clip 134 to a prior art packaging tray 136. Only a portion of the prior art packaging tray 136 containing a channel 142 in which the prior art retainer 130 is contained is shown. The "U" clips 134 snap over the prior art retainer 130 into grooves (not shown) in the prior art packaging tray 136 thereby securing the prior art retainer 130 to the prior art packaging tray 136. FIG 7A-1 illustrates a transverse cross section of the prior art packaging tray 136 and the "U" clip 134 taken along lines 7A-7A in FIG 7A. The "U" clip 134 has snaps 135 which snap into grooves 137 in the channel 148 wall. The prior art packaging tray 136 incorporating one "U" clip works as follows: As the catheter 152, illustrated in FIG 7A, is pulled away from the prior art packaging tray 136 the prior art retainer 130 slides through the "U" clip 134 until a flanged end 132 of said prior art retainer 130, which cannot fit through the "U" clip 134, is stopped by the "U" clip 134. At this point, since the prior art retainer 130 is prevented from moving in the direction of the pull, the balloon membrane (not shown because it is disposed within the prior art retainer 130) slides out of the proximal end of the prior art retainer 130. The prior art retainer 130 remains in the channel 142. If the prior art retainer 130, illustrated in FIG 7A, did not have a robust flanged end 132 then when the catheter 152 is pulled out of the prior art packaging tray 136 it would slide right through the "U" clip 134 and the prior art retainer 130 would remain disposed about the balloon membrane.

FIG 7B illustrates one of three PTFE tubes 85 which make up an improved retainer 82. Each tube 85 has an outer surface 88, a pair of lips 86, a first end 90, and a second end 92. Each tube 85 also has discontinuous wings 84 of various lengths which project from the outer surface 88 of the retainer 82 and lie between the first end 90 and the second end 92 of each tube 85. FIG 7C illustrates a cross section of retainer 82 taken along lines 7C-7C. Three tubes are used to facilitate advancing of the tubes: a number of shorter tubes are easier to advance while wrapping than one longer tube.

FIG 7D illustrates a plan view of an improved packaging tray 138 for a catheter 154, the improved retainer 82, and related accessories. Only the portion of the improved packaging tray 138 containing a channel 148 which contains the improved retainer 82 is shown. The wings 84 of the improved retainer 82 snap under horizontal ribs 146 in the channel 148 in the improved packaging tray 138. Vertical ribs 144 on both sides of the channel 148 fit between the retainer wings 84 and prevent the improved retainer 82 from being pulled out of the channel 148 while the catheter 152 is being removed from the improved packaging tray 138. Therefore, once the improved retainer 82 is snapped in place, the horizontal ribs 146 prevent vertical movement of the improved retainer 82 and the vertical ribs 144 prevent lateral movement of the improved retainer 82. Note that the outer diameter of the improved retainer 82, i.e. the distance from the end of one wing 84 to the end of its opposing wing 84, must be greater than the distance between opposing horizontal ridges 146.

FIG 7E illustrates a perspective view of one side of the channel 148 with the improved retainer 82 removed. The spacing and number of vertical ribs 144 and horizontal ribs 146 varies depending on the number of tubes 85 and the size of the balloon.

The co-lumen extruded tube 32, illustrated in FIG 2, is made of a blend of three medical polyurethane materials (medical polyurethane is the descriptive name of the material, it is sold under the name PELLETHANE) having the following weight percentages and hardness levels: 20% by weight of PELLETHANE having a hardness of 55D, 25% by weight of PELLETHANE having a hardness of 65D, and 55% by weight of PELLETHANE having a hardness of 75D. Use of this blend of materials yields a catheter demonstrating a high kink resistance. A high kink resistance is a very important feature for a catheter because catheter kinks block the lumen passageways and thus prevent inflation / deflation of the balloon and also create stress points which may lead to a catheter rupture. The co-lumen extruded tube 32 can be made from a number of different commercially available polyurethanes. The number of constituencies of the blend may vary from two to four, however, three is the optimal number of components. In order to insure the optimal stiffness and kink resistance at ambient and body temperature the components should fall within the following concentration ranges: 0-50% by weight of a softest component having a hardness between 80A and 55D; 30-60% by weight of a component having an intermediate hardness ranging between 100A and 65 D; and 0-70% by weight of a hardest component having a hardness between 55D and 80D. The material hardnesses should all fall within the range of 80A-80D.

The tip 40, as illustrated in FIG 2, is made from a biocompatible material, such as ESTANE material group 58887 (ESTANE is the trademark of the B.F. Goodrich chemical company and represents a thermoplastic urethane material (descriptive name)) and has a hardness not to exceed 90A. ESTANE is a softer material than the polyurethane used for the co-lumen extruded tube. A soft tip 40 made of ESTANE is desirable because it is flexible and thus will be able to follow the guide wire better than a harder tip. A second advantage to using ESTANE is that it is an elastomeric material which is very compatible with the balloon membrane material, thus allowing a good balloon membrane-to-tip bond. A third advantage to using a softer tip involves absorption of impact energy. If the distal end of a hard tip contacts the arterial wall during insertion, it will transfer a portion of its kinetic energy to the arterial wall and thereby deform the wall. If the transferred energy is above a certain critical level the tip will penetrate the arterial wall. A softer tip, on the contrary, will absorb a portion of the impact by deforming, and thereby, will decrease the likelihood of arterial wall penetration. Note that the stiffness of the soft tip can be increased by forming ribs, which extend from the proximal to the distal end of the tip, on the outer surface of the tip.

ESTANE material group 58887 is the ideal material for the tip because it demonstrates the desired hardness, extrusion properties, moldability, and hydrophilicity. Hydrophilicity measures the extent to which a film of water (as opposed to droplets) develops on the surface of a material immersed into water. In the case of a IAB catheter tip it is desired that a film (as opposed to droplets) develop on the surface of the tip.

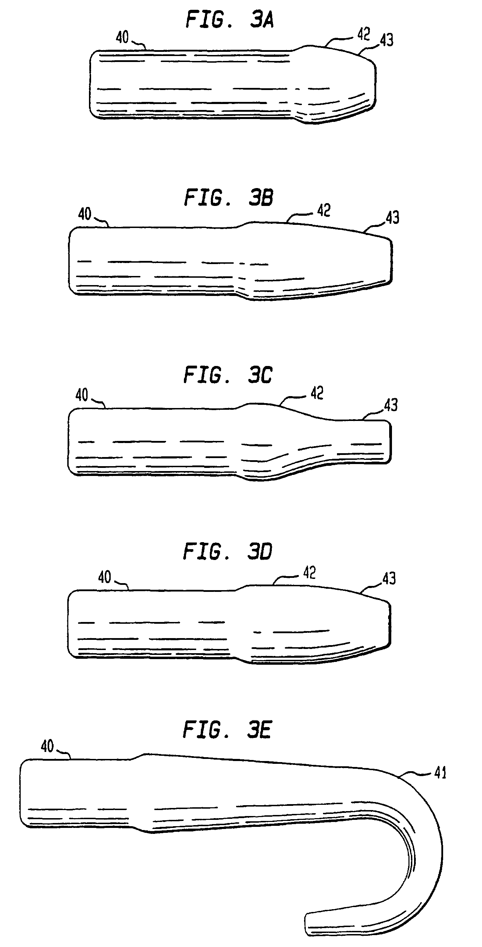

FIGS 3A, 3B, 3C, and 3D illustrate four different possible profiles for the tip 40. Each of the profiles have one common feature: a bulbous portion 42. Each tip has a distal end 43. The purpose of the bulbous portion 42, similar to the softer tip 40, is to facilitate guide wire tracking. A bulbous portion 42 distances the distal end 43 of the tip from the arterial wall and thus reduces scraping of the arterial wall by the distal end 43 of the tip 40 while the catheter is being guided through an especially tortuous portion of the aorta.

FIG 3E illustrates a fifth possible profile for the tip 40 having a hook portion 41. A tip having such a profile is less likely to damage the arterial wall during insertion because upon impact with a bend in the artery or with an obstruction the hook portion 41 will bend and absorb kinetic energy rather than remain stiff and impart all of its kinetic energy to the arterial wall.

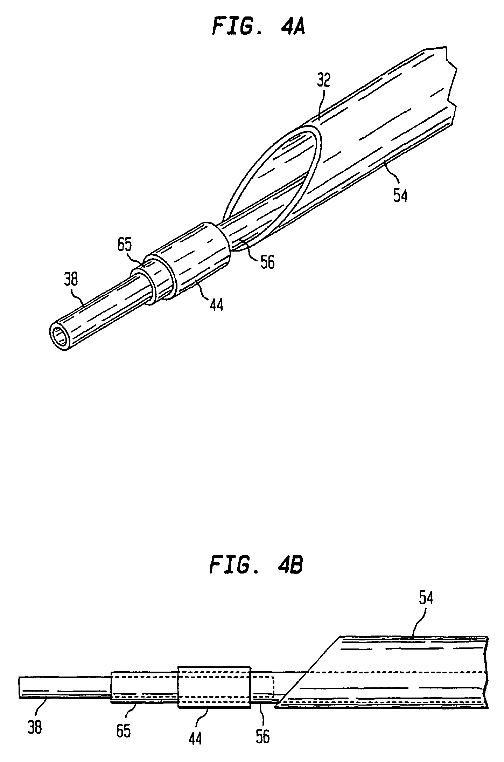

As noted earlier, Nitinol is only used for the Nitinol inner tube 38, rather than for an inner tube which would run the entire length of the catheter 30, because of the high price of Nitinol. Use of Nitinol only for the Nitinol inner tube 38, however, requires a means of attaching the inner tube portion 56 of the co-lumen extruded tube 32, made from polyurethane, to the Nitinol inner tube 38, made from Nitinol. FIG 4A illustrates a first method for creating a Nitinol-to-polyurethane bond. The catheter 30 illustrated in FIG 4A is shown without the balloon membrane 34. The first method involves:
1)coating the proximal end 70 of the Nitinol inner tube 38 with an adhesive, such as UV curable adhesive;
2)sliding the coated proximal end 70 of the Nitinol inner tube 38 into the distal end portion 65 of the inner tube portion 56 of the co-lumen extruded tube 32;
3) exposing the overlapping portions of the Nitinol inner tube 38 and the co-lumen extruded tube 32 to UV radiation in order to set the UV adhesive; and
4)crimping a metal radio-opaque marker 44 onto the distal end portion 65 of inner tube portion 56.
FIG 4B illustrates a longitudinal view of the Nitinol-to-plastic attachment point. Once again, the catheter 30 is shown without the balloon membrane 34.

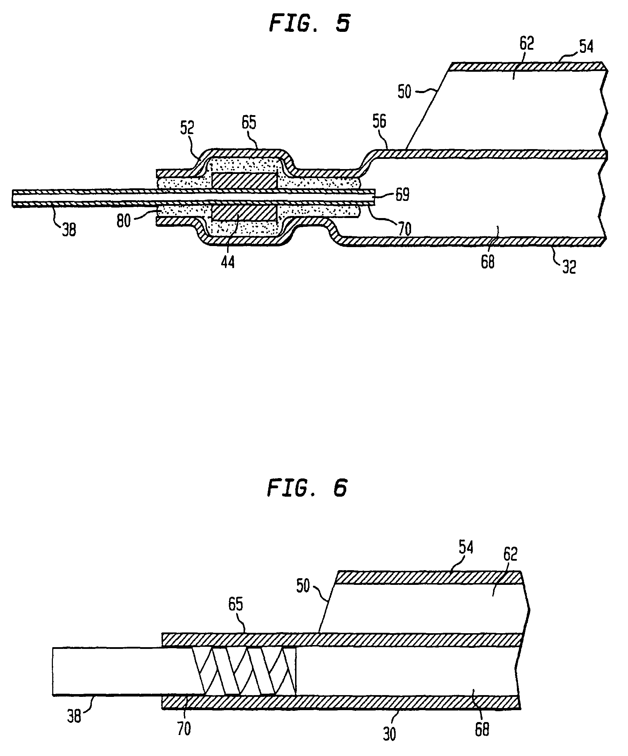

FIG 5 is a cross section of the catheter 30 taken along line A-A in FIG 2. FIG 5 illustrates a Nitinol-to-polyurethane bond created using a second method. The second method involves:
1)fastening a metal radio-opaque marker 44 to the proximal end 70 of the Nitinol inner tube 38;
2) coating the radio-opaque marker 44 and the portions of the Nitinol inner tube 38 just distal and proximal to the radio-opaque marker 44 with a UV adhesive 80;
3)inserting said proximal end 70 into the distal end portion 65 of the inner tube portion 56 of the co-lumen extruded tube 32;
4) exposing the coated portion of the Nitinol inner tube 38 to UV radiation so as to set the UV adhesive 80; and
5)heat setting the overlapping portions until the distal end portion 65 of the co-lumen extruded tube 32 conforms to the shape of the radio-opaque marker 44 and the Nitinol inner tube 38.

Once the Nitinol inner tube 38 and the inner tube portion 56 are attached, the catheter 30 may become too stiff in the attachment area. In order to reduce the stiffness of this area, resulting from the overlapping portions, a groove may be cut or inscribed in the proximal end 70 of the Nitinol inner tube 38, s illustrated in FIG 6. FIG 6 illustrates a longitudinal cross sectional view of the portion of the catheter 30 where the distal end portion 65 of the inner tube portion 56 is disposed about the proximal end 70 of the Nitinol inner tube 38. The radio-opaque marker 44 is not shown for clarity purposes.

## Claims

1. An intra-aortic balloon catheter (30) including an outer tube (54) with proximal and distal ends (48, 50), and an inner tube comprising a first inner tube (56) with proximal and distal (52) ends, and a balloon membrane (34) with proximal and distal ends (74, 76), the first inner tube (56) being disposed within an outer surface of the outer tube (54), the proximal end (74) of the balloon membrane (34) being connected to the distal end (50) of the outer tube (54), **characterized in that** said inner tube further comprises a second inner tube (38) with proximal and distal (70, 72) ends, and **in that** the second inner tube (38) is made of a super elastic metal alloy and extends beyond the distal end (50) of the outer tube (54), and **in that** the distal end (52) of the first inner tube (56) is attached to the proximal end (70) of the second inner tube (38), and **in that** the distal end (76) of the balloon membrane (34) is connected to the distal end (72) of the second inner tube (38).

2. The intra-aortic balloon catheter (30) as claimed in claim 1 wherein the super elastic metal alloy is Nitinol.

3. An intra-aortic balloon catheter as claimed in claim 1 or 2, wherein the first inner tube (56) and the outer tube (54) together comprise a co-lumen extruded tube (32).

4. An intra-aortic balloon catheter as claimed in claim 3, wherein an inner lumen passageway (57) exists inside the first inner tube (56) and extends from a proximal end of the co-lumen extruded tube (32) to a second distal end (52) of the co-lumen extruded tube (32), an outer lumen passageway (62) is defined by the inner surface of the outer tube (54) and extends from the proximal end of the co-lumen extruded tube (32) to a first distal end (50) of the co-lumen extruded tube (32), the inner lumen and outer lumen passageways (57, 62) are partially defined by a common portion of the co-lumen extruded tube (32).

5. An intra-aortic balloon catheter as claimed in claim 4, wherein the second distal end (52) of the co-lumen extruded tube (32) is further distal than the first distal end (50) of the co-lumen extruded tube (32), the inner lumen passageway (57) extends beyond the first distal end (50) of the co-lumen extruded tube (32) and is longer than the outer lumen passageway (62), the proximal end (74) of the balloon membrane (34) is connected to the first distal end (50) of the co-lumen extruded tube (32) and the distal end (76) of the balloon membrane (34) is connected to the distal end (72) of the second inner tube (38).

6. An intra-aortic balloon catheter as claimed in any one of the preceding claims, comprising a non-stick coating on the balloon membrane (34) outer surface which decreases the coefficient of friction of the balloon membrane surface prior to insertion into a patient.

7. An intra-aortic balloon catheter as claimed in claim 6, wherein the non-stick coating is medical silicone.

8. An intra-aortic balloon catheter as claimed in any one of the preceding claims, wherein the second inner tube (38) has an inner surface and an outer surface, and wherein a portion of the inner tube just distal to the proximal end has a spiral cut extending from the outer surface to the inner surface.

9. An intra-aortic balloon catheter as claimed in any one of the preceding claims, further comprising a radio-opaque marker (44) crimped to the second inner tube (38) at a location where the second inner tube (38) is attached to the distal end (52) of the first inner tube (56).

## Patentansprüche

1. Intra-aortaler Ballonkatheter (30), aufweisend ein äußeres Rohr (54) mit proximalem und distalem Ende (48, 50) und ein inneres Rohr, aufweisend ein erstes inneres Rohr (56) mit einem proximalen und einem distalen (52) Ende und eine Ballonmembran (34) mit einem proximalen und einem distalen Ende (74, 76), wobei das erste innere Rohr (56) innerhalb einer Außenoberfläche des äußeren Rohres (54) angeordnet ist, wobei das proximale Ende (74) der Ballonmembran (34) verbunden ist mit dem distalen Ende (50) des äußeren Rohres (54), **dadurch gekennzeichnet, daß** das innere Rohr des weiteren ein zweites inneres Rohr (38) mit einem proximalen und einem distalen Ende (70, 72) aufweist und daß das zweite innere Rohr (38) aus einer superelastischen Metallegierung hergestellt ist und sich über das distale Ende (50) des äußeren Rohres (54) erstreckt und daß das distale Ende (52) des ersten inneren Rohres (56) an dem proximalen Ende (70) des zweiten inneren Rohres (38) angebracht ist und daß das distale Ende (76) der Ballonmembran (34) mit dem distalen Ende (72) des zweiten inneren Rohrs (38) verbunden ist.

2. Intra-aortaler Ballonkatheter (30) nach Anspruch 1, bei dem die superelastische Metallegierung Nitinol ist.

3. Intra-aortaler Ballonkatheter nach Anspruch 1 oder 2, bei welchem das erste innere Rohr (56) und das äußere Rohr (54) zusammen ein ko-lumenartiges, extrudiertes Rohr (32) umfassen.

4. Intra-aortaler Ballonkatheter nach Anspruch 3, bei dem ein Innenlumendurchgang (57) im Inneren des ersten inneren Rohrs (56) besteht und von einem proximalen Ende des ko-lumenartigen, extrudierten Rohres (32) zu einem zweiten distalen Ende (52) des ko-lumenartigen, extrudierten Rohres (32) verläuft, ein Außenlumendurchgang (62) begrenzt ist durch die Innenoberfläche des äußeren Rohrs (54) und von dem proximalen Ende des ko-lumenartigen, extrudierten Rohres (32) zu einem ersten distalen Ende (50) des ko-lumenartigen, extrudierten Rohres (32) verläuft, wobei der Innenlumen- und der Außenlumendurchgang (57, 62) teilweise begrenzt sind durch einen gemeinsamen Abschnitt des ko-lumenartigen, extrudierten Rohrs (32).

5. Intra-aortaler Ballonkatheter nach Anspruch 4, bei dem das zweite distale Ende (52) des ko-lumenartigen, extrudierten Rohres (32) weiter distal liegt als das erste distale Ende (50) des ko-lumenartigen, extrudierten Rohres (32), der Innenlumendurchgang (57) sich über das erste distale Ende (50) des ko-lumenartigen, extrudierten Rohres (32) erstreckt und länger ist als der Außenlumendurchgang (62), das proximale Ende (74) der Ballonmembran (34) mit dem ersten distalen Ende (50) des ko-lumenartigen, extrudierten Rohres (32) verbunden ist und das distale Ende (76) der Ballonmembran (34) mit dem distalen Ende (72) des zweiten inneren Rohres (38) verbunden ist.

6. Intra-aortaler Ballonkatheter nach einem der vorstehenden Ansprüche, aufweisend eine nicht-haftende Beschichtung auf der Außenoberfläche der Ballonmembran (34), welche den Reibungskoeffizienten der Oberfläche der Ballonmembran vor dem Einführen in einen Patienten mindert.

7. Intra-aortaler Ballonkatheter nach Anspruch 6, bei dem die nicht-haftende Beschichtung medizinisches Silikon ist.

8. Intra-aortaler Ballonkatheter nach einem der vorstehenden Ansprüche, bei dem das zweite innere Rohr (38) eine Innenoberfläche und eine Außenoberfläche hat und wobei ein Abschnitt des inneren Rohres, unmittelbar distal des proximalen Endes, einen spiralförmigen Schnitt hat, der von der Außenoberfläche zu der Innenoberfläche verläuft.

9. Intra-aortaler Ballonkatheter nach einem der vorstehenden Ansprüche, des weiteren aufweisend einen strahlenundurchlässigen Marker (44), der angecrimpt ist an dem zweiten inneren Rohr (38) an einer Stelle, wo das zweite innere Rohr (38) an dem distalen Ende (52) des ersten inneren Rohres (56) befestigt ist.

## Revendications

1. Cathéter à ballonnet intra-aortique (30) comprenant un tube extérieur (54) muni d'une extrémité proximale et d'une extrémité distale (48, 50), et un tube intérieur comprenant un premier tube intérieur (56) muni d'une extrémité proximale et d'une extrémité distale (52), et une membrane de ballonnet (34) possédant une extrémité proximale et une extrémité distale (74, 76), le premier tube intérieur (56) étant disposé dans une surface extérieure du tube extérieur (54), l'extrémité proximale (74) de la membrane de ballonnet (34) étant reliée à l'extrémité distale (50) du tube extérieur (54), **caractérisé en ce que** ledit tube intérieur comprend en outre un second tube intérieur (38) possédant une extrémité proximale et une extrémité distale (70, 72), **en ce que** le second tube intérieur (38) est constitué d'un alliage métallique super élastique et s'étend au-delà de l'extrémité distale (50) du tube extérieur (54), **en ce que** l'extrémité distale (52) du premier tube intérieur (56) est reliée à l'extrémité proximale (70) du second tube extérieur (38), et **en ce que** l'extrémité distale (76) de la membrane de ballonnet (34) est reliée à l'extrémité distale (72) du second tube intérieur (38).

2. Cathéter à ballonnet intra-aortique (30) selon la revendication 1, dans lequel l'alliage métallique super élastique est du Nitinol.

3. Cathéter à ballonnet intra-aortique selon la revendication 1 ou 2, dans lequel le premier tube intérieur (56) et le tube extérieur (54) comprennent ensemble un tube extrudé à lumière commune (32).

4. Cathéter à ballonnet intra-aortique selon la revendication 3, dans lequel un passage de lumière intérieur (57) existe à l'intérieur du premier tube intérieur (56) et s'étend d'une extrémité proximale du tube extrudé à lumière commune (32) à une seconde extrémité distale (52) du tube extrudé à lumière commune (32), un passage de lumière extérieur (62) est défini par la surface intérieure du tube extérieur (54) et s'étend de l'extrémité proximale du tube extrudé à lumière commune (32) à une première extrémité distale (50) du tube extrudé à lumière commune (32), et les passages de lumière intérieur et extérieur (57, 62) sont partiellement définis par une portion commune du tube extrudé à lumière commune (32).

5. Cathéter à ballonnet intra-aortique selon la revendication 4, dans lequel la seconde extrémité distale (52) du tube extrudé à lumière commune (32) est plus distale que la première extrémité distale (50) du tube extrudé à lumière commune (32), le passage de lumière intérieur (57) s'étend au-delà de la première extrémité distale (50) du tube extrudé à lumière commune (32) et est plus long que le passage de lumière extérieur (62), l'extrémité proximale (74) de la membrane de ballonnet (34) est reliée à la première extrémité distale (50) du tube extrudé à lumière commune (32) et l'extrémité distale (76) de la membrane de ballonnet (34) est reliée à l'extrémité distale (72) du second tube intérieur (38).

6. Cathéter à ballonnet intra-aortique selon l'une quelconque des revendications précédentes, comprenant un revêtement non collant sur la surface extérieure de la membrane de ballonnet (34) qui réduit le coefficient de frottement de la surface de la membrane de ballonnet avant l'insertion dans un patient.

7. Cathéter à ballonnet intra-aortique selon la revendication 6, dans lequel le revêtement non collant est un silicone médical.

8. Cathéter à ballonnet intra-aortique selon l'une quelconque des revendications précédentes, dans lequel le second tube intérieur (38) possède une surface intérieure et une surface extérieure, et dans lequel une portion du tube intérieur juste distale par rapport à l'extrémité proximale possède une découpe en spirale s'étendant de la surface extérieure à la surface intérieure.

9. Cathéter à ballonnet intra-aortique selon l'une quelconque des revendications précédentes, comprenant en outre un marqueur opaque aux radiations (44) se conformant au second tube intérieur (38) à un emplacement auquel le second tube intérieur (38) est relié à l'extrémité distale (52) du premier tube intérieur (56).
